(19) Europäisches Patentamt
European Patent Office
Office européen des brevets

(11) **EP 3 662 821 A1**

(12) **EUROPEAN PATENT APPLICATION**

(43) Date of publication:
**10.06.2020 Bulletin 2020/24**

(51) Int Cl.:
***A61B 5/021*** (2006.01)   ***A61B 5/026*** (2006.01)
***A61B 5/00*** (2006.01)

(21) Application number: **18209838.4**

(22) Date of filing: **03.12.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **PRESURA, Cristian Nicolae**
  **5656 AE Eindhoven (NL)**
• **AARTS, Ronaldus Maria**
  **5656 AE Eindhoven (NL)**
• **MUEHLSTEFF, Jens**
  **5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE FOR USE IN MEASURING BLOOD PRESSURE**

(57)   There is provided a device (100) for use in measuring blood pressure. The device (100) comprises a light source (102) configured to emit light (104) onto the skin (106) of a subject (108). The light source (102) is configured to alternate between emitting coherent light and incoherent light. The device (100) also comprises a light detector (110) configured to detect light (104) scattered back from the skin (106) of the subject (108) and generate an electrical signal from the detected light. The generated electrical signal is for use in measuring a blood pressure of the subject (108).

FIG. 1

**Description**

FIELD OF THE INVENTION

The disclosure relates to a device for use in measuring blood pressure and a method of operating the device.

BACKGROUND OF THE INVENTION

Blood pressure (BP), or more specifically arterial blood pressure (ABP), is considered to be one of the most important physiological parameters for monitoring subjects. This is especially the case for subjects in a critical condition. Blood pressure can, for example, provide a useful indication of the physical or physiological condition of a subject. It is thought of as a key physiological parameter relevant for medical diagnosis, prevention, as well as therapy guidance.

A variety of techniques exist for measuring blood pressure. The techniques can be divided into two categories, namely invasive techniques and non-invasive techniques. The invasive techniques currently provide the most accurate continuous measurements. However, most invasive techniques are expensive and are generally only used in specific settings such as hospitals (especially in intensive care units) as they require specialists or trained medical staff for operation. Therefore, non-invasive techniques can be more practical where blood pressure measurements need to be acquired more frequently by an untrained user. Due to the availability of affordable devices, cuff-based blood pressure measurements have found widespread use in home monitoring.

However, standard cuff-based blood pressure measurements that use the widely known oscillometry or auscultation methods only allow intermittent measurements and are uncomfortable for the subject. In particular, in the standard cuff-based blood pressure measurements, the pressure of the cuff needs to be close to or higher than the diastolic blood pressure (around 80 mm-Hg) for blood pressure measurement, whereas comfortable pressures are much lower (e.g. with a maximum in the range from 20-30 mmHg).

Other techniques that have been seen as promising technologies towards continuous blood pressure measurements include radial artery applanation tonometry, vascular unloading and pulse wave velocity (PWV) or pulse shape analysis techniques. However, each of these techniques also have their downsides. Radial artery tonometry, for example, is inherently sensitive to sensor displacement and motion artifacts, which makes it unsuitable for wearable devices. Also, in the case of vascular unloading, for example, a cuff still needs to be placed on the finger of the subject and the placement of this cuff in vascular unloading is difficult and cumbersome. Also, absolute blood pressure readings can be unreliable despite an underlying calibration scheme and the overall system is large and bulky.

WO 2007/017661 discloses another technique for measuring blood pressure, which also involves a cuff to apply pressure. In this technique, coherent light is launched into the skin of the subject at a point downstream of the cuff and light scattered back from the skin of the subject is processed to create a signal indicative of a level of blood flow oscillation from which an arterial blood pressure can be determined. However, when an artery is stiff, the volume of the artery will not change much with blood pressure and thus the level of blood flow oscillation is not always a reliable indicator of the blood pressure. An optical pulse signal (which provides an indication of the volume) is thus not adequate to measure absolute values of arterial blood pressure at low pressures of the cuff (or in the absence of the cuff).

Some techniques have been proposed to address this by taking into account the stiffness of the artery or a correlated parameter. One example of such a technique involves ultrasound to measure two parameters of an artery at the same time, namely the volume of the artery (and its pulse variation) and the velocity of the blood in that artery. When the artery is stiff, the blood pressure increase does not significantly increase the artery volume, but does increases the velocity of the blood flowing in the artery. On the other hand, when the artery is not stiff, the volume of the artery changes more, and the velocity less. The technique assumes that the local speed of the blood flow in the artery is correlated to the stiffness of the artery. Thus, the stiffness of the artery can be taken into account by using the local speed of the blood flow in the artery in the determination of a local blood pressure measurement.

However, although this technique works for measuring blood pressure locally with only a very small pressure of cuff (or no cuff at all), the ultrasound equipment used to measure the signals above an artery is very cumbersome, large, and not portable. As such, the equipment cannot be integrated into a wearable device.

SUMMARY OF THE INVENTION

As noted above, a limitation associated with existing techniques is that it is not currently possible to accurately and continuously measure blood pressure of a subject without applying large (uncomfortable) pressures with a cuff or without the use of cumbersome, large, and not portable equipment. It would thus be valuable to address this limitation and provide an improved device for use in measuring blood pressure.

Therefore, according to a first aspect, there is provided a device for use in measuring blood pressure. The device comprises a light source configured to emit light onto a portion of the skin of a subject and a light detector configured to detect light scattered back from said portion of the skin of the subject and generate an electrical signal from the detected light. The light source is configured to alternate between emitting coherent light and incoherent light and the generated electrical signal is for use in measuring a blood pressure of the subject.

In some embodiments, the light source may be configured to alternate between emitting coherent light and incoherent light when a current of the light source is altered.

In some embodiments, the light source may be configured to emit coherent light when the current of the light source is increased above a threshold current and/or emit incoherent light when the current of the light source is decreased below the threshold current. In some embodiments, the light source may be configured to emit coherent light when the current of the light source is increased to a current that is a predefined amount greater than the threshold current and/or emit incoherent light when the current of the light source is decreased to a current that is the predefined amount less than the threshold current.

In some embodiments, the light source may be configured to alternate between emitting coherent light and incoherent light with a predefined frequency.

In some embodiments, the electrical signal generated when the light source is configured to emit coherent light may be for use in measuring a blood flow in the skin of the subject.

In some embodiments, the light source may be configured to operate as a light emitting diode to emit incoherent light. In some embodiments, the light source may be configured to operate as a laser to emit coherent light.

In some embodiments, the device may further comprise an actuator configured to maintain a pressure with which the device is applied to the skin of the subject at a predefined pressure.

According to a second aspect, there is provided a system for use in measuring blood pressure. The system comprises the device as described earlier. The system may also comprise a processor. In some embodiments, the processor may be configured to process the electrical signal generated when the light source alternates between emitting coherent light and incoherent light to measure the blood pressure of the subject and/or process the electrical signal generated when the light source emits coherent light to measure the blood flow in the skin of the subject.

In some embodiments, processor may be configured to measure the blood pressure of the subject by being configured to determine a pulse amplitude of the electrical signal generated when the light source emits incoherent light, determine a pulse wave velocity of the electrical signal generated when the light source emits coherent light, and measure the blood pressure of the subject based on the determined pulse amplitude and the determined pulse wave velocity.

In some embodiments, the processor may be configured to measure the blood flow in the skin of the subject by being configured to invert the electrical signal generated when the light source emits coherent light, determine a maximum frequency of the inverted electrical signal, and measure the blood flow in the skin of the subject as an average velocity of blood flow in the skin of the subject. In some embodiments, the average velocity of blood flow in the skin of the subject may be half of a product of the determined maximum frequency of the inverted electrical signal and a wavelength at which the coherent light is emitted.

According to a third aspect, there is provided a method of operating a device as described earlier. The method comprises emitting the light onto a portion of the skin of the subject, alternating the light source between emitting coherent light and incoherent light, detecting the light scattered back from within the portion of the skin of the subject, and generating the electrical signal from the detected light.

In some embodiments, the method may further comprise processing the electrical signal generated when the light source alternates between emitting coherent light and incoherent light to measure the blood pressure of the subject and/or processing the electrical signal generated when the light source emits coherent light to measure the blood flow in the skin of the subject.

According to a fourth aspect, there is provided a computer program product comprising a computer readable medium, the computer readable medium having a computer readable code embodied therein, the computer readable code being configured such that, on execution by a suitable computer or processor, the computer or processor is caused to perform the method described above.

According to the aspects and embodiments described above, the limitation associated with existing techniques is addressed. In particular, the above-described aspects and embodiments, it is possible to generate an electrical signal from which a more accurate and reliable blood pressure measurement can be determined. It is possible to generate this electrical signal without the need for the application of high pressures by a wearable cuff and without the need for cumbersome equipment. Instead, the device is less complex and can be made portable and is more comfortable in use. The device is also suitable for continuous monitoring applications. In this way, there is provided an improved device for use in measuring blood pressure. The limitation associated with existing techniques is therefore addressed by way of the above-described aspects and embodiments.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a simplified schematic illustration of a device for use in measuring blood pressure according to an embodiment;
Fig. 2 illustrates a current of a light source of the device according to an embodiment;
Fig. 3 is a simplified schematic illustration of a device for use in measuring blood pressure according to another embodiment;
Fig. 4 illustrates a current of a light source of the device according to an embodiment;
Fig. 5 is a simplified schematic illustration of a device for use in measuring blood pressure according to another embodiment and a current of a light source of the device according to an embodiment;
Fig. 6 is a simplified schematic illustration of a device for use in measuring blood pressure according to another embodiment and a current of a light source of the device according to an embodiment; and
Fig. 7 is a flow chart illustrating a method of operating a device for use in measuring blood pressure according to an embodiment.

DETAILED DESCRIPTION OF EMBODIMENTS

There is described herein a device for use in measuring blood pressure. The device can be for use in measuring blood pressure via photoplethysmography (PPG). The device can be configured to be worn by a subject. Thus, the device can be a wearable device. In some embodiments, the device may comprise a strap (or band) for placement around a part of the body of the subject, such that the device can be worn at a location suitable for measuring blood pressure.

The device may be configured to be worn by a subject at any location on the body of the subject suitable for measuring blood pressure. In some embodiments, the location may be a location comprising bone close to the skin surface (e.g. to ensure a well-defined contact pressure). For example, in some embodiments, the device can be configured to be worn on a finger of a subject, a forehead of a subject, a wrist of a subject, a chest (e.g. the center of the chest) of a subject, or any other location on the body of the subject suitable for measuring blood pressure.

The subject can be any type of subject (e.g. a patient or any other subject). The device can be configured for any purpose. For example, the device can be a medical device according to some embodiments or a non-medical device according to other embodiments. The device can be for use by a trained user, such as a medical professional (e.g. a doctor, a nurse, or any other medical professional) or an untrained user (e.g. the subject themselves, a care giver, a family member, or any other untrained user). The device can be for use in a medical environment (e.g. a hospital, a surgery, or any other medical environment) and/or non-medical environment (e.g. at home, or any other non-medical environment).

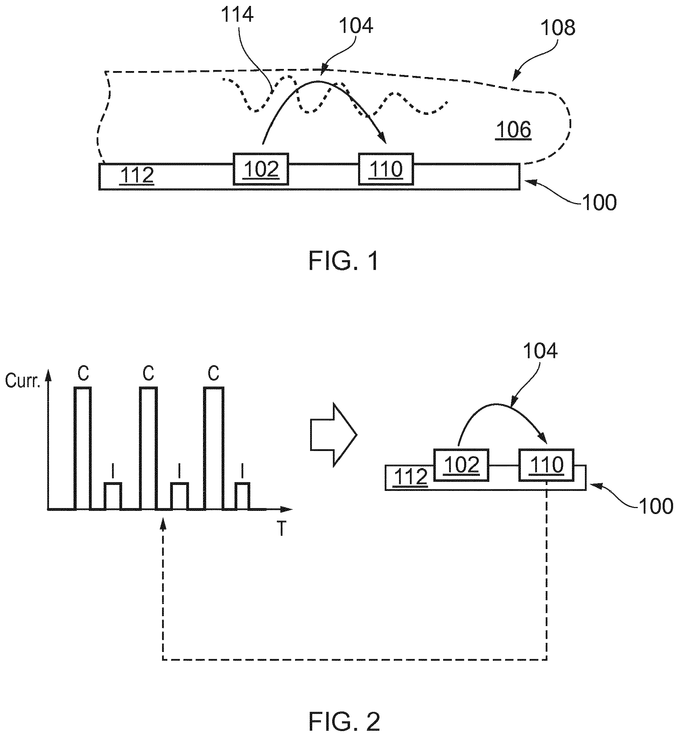

Fig. 1 illustrates a device 100 for use in measuring blood pressure according to an embodiment. As illustrated in Fig. 1, the device 100 comprises a light source 102. The light source 102 is configured to emit light 104 onto the skin 106 of a subject 108. The light source 102 is configured to alternate between emitting coherent light and incoherent light. In some embodiments, the light source 102 is configured to alternate between emitting coherent light and incoherent light when a current of the light source 102 is altered. The device 100 also comprises a light detector 110, e.g. a photodetector. The light detector 110 is configured to detect light 104 scattered back from (which may also include passing through a portion of) the skin 106 of the subject 108 and generate an electrical signal from the detected light 104. The generated electrical signal is for use in measuring a blood pressure of the subject 108.

As illustrated in Fig. 1, in use, the device 100 is applied to the skin 106 of the subject 108. For example, in use, the device 100 can be in contact with the skin 106 of the subject 108. In some embodiments, the light source 102 and/or the light detector 110 can be positioned such that, in use, the light source 102 and/or the light detector 110 are applied to (e.g. in contact with) the skin 106 of the subject 108. As illustrated in Fig. 1, in some embodiments, the device 100 can comprise a supporting member (or base) 112. In some of these embodiments, the light source 102 and/or the light detector 110 can be positioned on the supporting member 112. In use, the supporting member 112 may be applied to (e.g. in contact with) the skin 106 of the subject 108.

Although not illustrated in Fig. 1, in some embodiments, the device 100 may comprise a cover through which the light source 102 is configured to emit light 104 onto the skin 106 of a subject 108 and through which the light detector 110 is configured to detect light 104 scattered back from the skin 106 of the subject 108. In these embodiments, the cover may be applied to (e.g. in contact with) the skin 106 of the subject 108. The cover can, for example, be transparent or at least partially transparent.

In the embodiment illustrated in Fig. 1, the skin 106 of the subject 108 is the skin 106 of a finger of the subject 108. However, it will be understood that the skin 106 may be of any other body part of the subject 108 suitable for use in measuring blood pressure. The light 104 emitted from the light source 102 onto the skin 106 of the subject 108 is illustrated in Fig. 1 with reference to an artery 114. As illustrated in Fig. 1, the light 104 can pass through a portion of the skin, which includes the artery 114. Thus, the light detector 110 can detect light 104 scattered back from the artery located within said portion of the skin 106 of the subject 108. However, it will be understood that the device 100 may alternatively only be scattered back from a superficial layer of the skin 106. The incoherent light can travel via the same path from the light source 102 to the light detector 110 as the coherent light and vice versa. In this way, any motion artefacts in the electrical signal generated from the detected light 104 will be consistent.

Fig. 2 illustrates the current of the light source 102 of the device 100 according to the embodiment illustrated in Fig. 1. In particular, as illustrated in Fig. 2, the light source 102 of the device 100 illustrated in Fig. 1 can be configured to alternate between emitting coherent light ("C") and incoherent light ("I") when a current ("Curr.") of the light source 102 is altered. The current of the light source 102 is altered over time ("T").

As illustrated in Fig. 2, in some embodiments, a feedback loop may be used from the light detector 110 to the current of the light source 102. Thus, in some embodiments, the current of the light source 102 may be controlled based on feedback received from the light detector 110. In some embodiments, for example, the feedback received from the light detector 110 may be used to adjust one or more properties of the current control, such as current control intensities, duty cycle, etc. In this way, it can be ensured that the optimal properties are used for current control, e.g. to take into account that properties of the skin can be different. Thus, the device 100 can be appropriately calibrated.

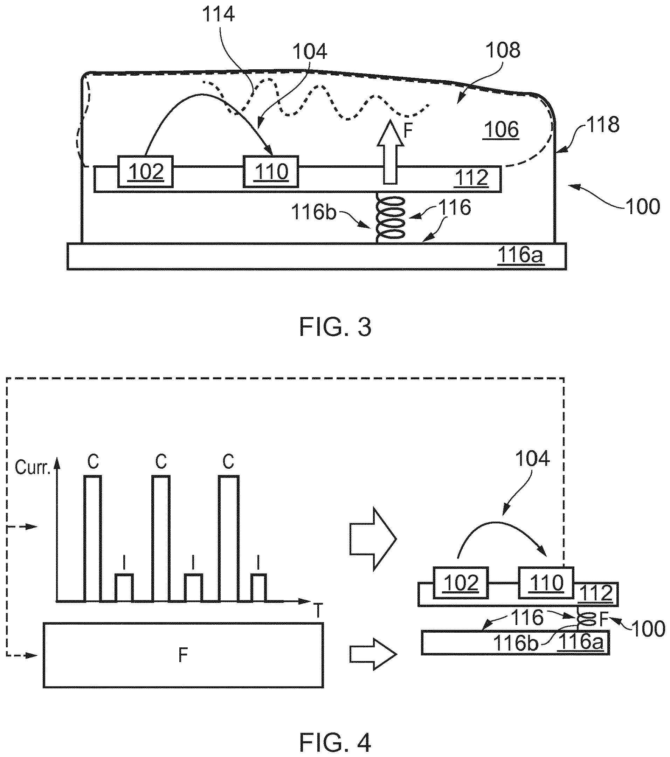

Fig. 3 is a simplified schematic illustration of the device 100 for use in measuring blood pressure according to another embodiment. In the embodiment illustrated in Fig. 3, the device 100 is as described earlier with respect to Fig. 1. In addition, in the embodiment illustrated in Fig. 3, the device 100 further comprises an actuator 116. The actuator 116 can be configured to apply pressure to the skin 106 of the subject 108. For example, the actuator 116 can be configured to cause the device 100 (e.g. any one or more of the light source 102, the light detector 110, a supporting member 112, and a cover of the device) to apply pressure to the skin 106 of the subject 108 in use. The actuator 116 may be positioned such that, in use, the pressure is applied to the skin 106 of the subject 108 at a local point for the measurement of blood pressure. Fig. 3 illustrates the force F with which the device 100 can apply pressure to the skin 106 of the subject 108. In some embodiments, the actuator 116 can comprise a mechanical actuator.

In some embodiments, as illustrated in Fig. 3, the actuator 116 may comprise a moveable member 116a. The moveable member 116a can be configured to move toward the skin 106 of the subject 108 (e.g. in the direction of the force F) in use to apply pressure on the skin 106. For example, in embodiments where the device 100 comprises the supporting member 112, the moveable member 116a can be configured to move toward the supporting member 112 in use to cause the supporting member 112 to apply pressure on the skin 106 (e.g. directly and/or via any one or more of the light source 102, the light detector 110, and a cover of the device).

In some embodiments, as illustrated in Fig. 3, the actuator 116 may comprise a tension member (e.g. a spring) 116b. For example, in embodiments where the device 100 comprises the supporting member 112 and the moveable member 116a, the tension member 116b may be positioned between the supporting member 112 and the moveable member 116a. The tension member 116b can be configured to push the supporting member 112 (and thus, for example, any one or more of the light source 102, the light detector 110 and a cover) of the device 100 into the skin. As illustrated in Fig. 3, in some embodiments where the device 100 comprises a strap (or band) 118 for placement around a part of the body of the subject 108, the moveable member 116a may be connected to the strap 118.

As mentioned earlier, the actuator 116 can be configured to apply pressure to the skin 106 of the subject 108. More specifically, the actuator 116 can be configured to maintain a pressure with which the device 100 (e.g. any one or more of the light source 102, the light detector 110, the supporting member 112, and the cover of the device) is applied to the skin 106 of the subject 108 at a predefined pressure. For example, the actuator 116 can ensure that a constant pressure is applied by the device 100 (e.g. any one or more of the light source 102, the light detector 110, a supporting member 112, and a cover of the device) to the skin 106 of the subject 108 when the device 100 is in use. In some embodiments, the device 100 may be calibrated before use and the predefined pressure may be the pressure used for calibration. The predefined pressure is 'predefined' in that it is a set (or known) value. In some embodiments, the predefined pressure may be a pressure of less than 30 mm-Hg. Thus, blood pressure can be measured using the device 100 even at low (comfortable) pressures. In some embodiments, the predefined pressure may be changed to enable more reliable measurements of blood pressure.

The use of the actuator 116 to ensure the pressure applied to the skin 106 of the subject 108 is maintained at the predefined pressure provides a more reliable device 100, since any variations in pressure can be compensated in this way. These variations in pressure may otherwise create a corresponding variation in the electrical signal generated from the detected light 104. For example, the variation may be in a direct current (DC) component of the generated electrical signal. If the pressure applied to the skin 106 of the subject 108 increases, the DC component of the generated electrical signal may also increase since some blood is pushed away by the increased pressure applied to the skin 106. Similarly, for example, the variation may be in an average velocity of blood flow in the skin 106 measured from the generated electrical signal. Any variation in the electrical signal generated from the detected light 104 may thus cause a variation in blood pressure and/or blood flow measured from the generated electrical signal. However, by use of the actuator 116 to ensure the pressure applied to the skin 106 of the subject 108 is maintained at the predefined pressure, these variations can be avoided. The device 100 can thus be for use in measurement of a continuous blood pressure form. In effect, the actuator 116 functions like a calibration point.

Fig. 4 illustrates the current of the light source 102 of the device 100 according to the embodiment illustrated in Fig. 3. In particular, as illustrated in Fig. 4, the light source 102 of the device 100 illustrated in Fig. 3 can be configured to alternate between emitting coherent light ("C") and incoherent light ("I") when a current ("Curr.") of the light source 102 is altered. The current of the light source 102 is altered over time ("T").

As illustrated in Fig. 4, in some embodiments, a feedback loop may be used from the light detector 110 to the current of the light source 102. Thus, in some embodiments, the current of the light source 102 may be controlled based on feedback received from the light detector 110. In some embodiments, for example, the feedback received from the light detector 110 may be used to adjust one or more properties of the current control, such as current control intensities, duty cycle, etc. In this way, it can be ensured that the optimal properties are used for current control, e.g. to take into account that properties of the skin can be different. Thus, the device 100 can be appropriately calibrated.

Alternatively or in addition, in some embodiments, a feedback loop may be used from the light detector 110 to the force F with which the actuator 116 applies pressure to the skin 106 of the subject 108. Thus, in some embodiments, the force F with which the actuator 116 applies pressure to the skin 106 of the subject 108 may be controlled based on feedback received from the light detector 110. In some embodiments, for example, the feedback received from the light detector 110 may be used to adjust the force F with which the actuator 116 applies pressure to the skin 106 of the subject 108. For example, the predefined pressure at which the pressure applied to the skin 106 of the subject 108 is maintained may be adjusted based on the feedback received from the light detector 110. In this way, the device 100 can be appropriately calibrated.

As mentioned earlier and as illustrated in Figs. 2 and 4, in the embodiments described with reference to Figs. 1-4, the light source 102 of the device 100 is configured to alternate between emitting coherent light and incoherent light and can be configured to alternate in this way when a current of the light source 102 is altered. In some embodiments, the light source 102 can be configured to emit coherent light when the current of the light source 102 is increased above a threshold current. The threshold current can depend on the type of the light source 102. In some embodiments, the threshold current can be few milliampere (mA). Alternatively or in addition, in some embodiments, the light source 102 can be configured to emit incoherent light when the current of the light source 102 is decreased below the threshold current.

In some of these embodiments, the light source 102 may be configured to emit coherent light when the current of the light source is increased to a current that is a predefined amount greater than the threshold current. Alternatively or in addition, in some embodiments, the light source 102 may be configured to emit incoherent light when the current of the light source 102 is decreased to a current that is the predefined amount less than the threshold current. In some embodiments, the predefined amount can be a predefined percentage. For example, the predefined percentage may be 10%, 20%, 30%, 40% or 50% (e.g. 30%). In some embodiments, predefined percentage may be selected based on one or more operational requirements, e.g. a low power consumption and/or high quality.

In some embodiments, a predetermined set of values may instead be used for the current control. For example, the predetermined set of values may comprise a first predetermined value for the current of the light source 102 to be configured to emit incoherent light and a second predetermined value for the current of the light source 102 to be configured to emit coherent light. In some embodiments, the first predetermined value may be 30 mA and the second threshold value may be 60 mA.

In the embodiments illustrated in Figs. 1-4, the light source 102 of the device 100 comprises a single light source that is configured to alternate between emitting coherent light and incoherent light. This ensures that the coherent light and the incoherent light travels through the same part of skin 106, which can provide more accurate blood pressure measurements. Nevertheless, it will be understood that, in other embodiments, the light source 102 of the device 100 may comprise a plurality of (e.g. separate) light sources that are configured to alternate between emitting coherent light and incoherent light. In these embodiments, the coherent light and the incoherent light may travel through different parts of skin 106.

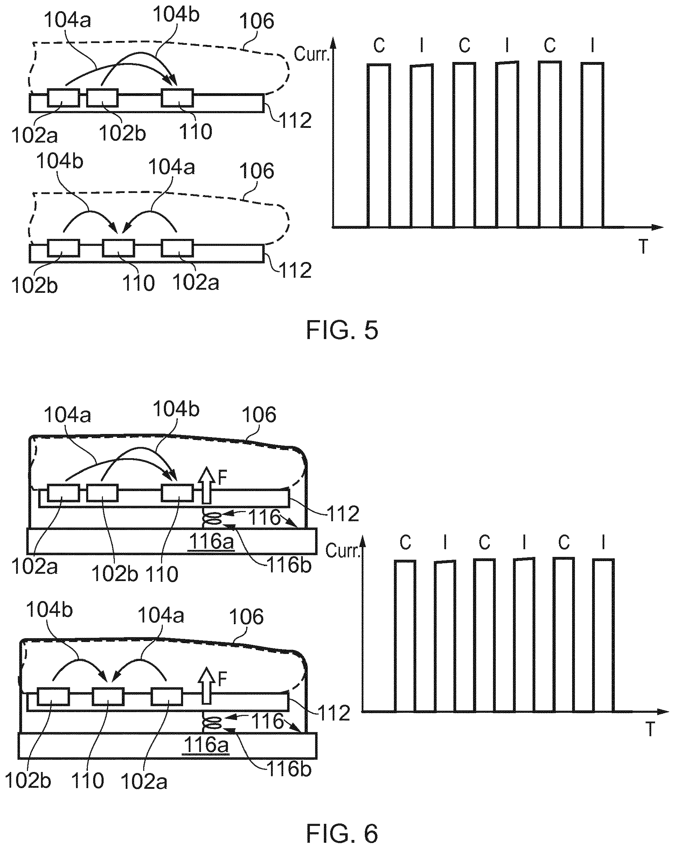

Fig. 5 is a simplified schematic illustration of the device 100 for use in measuring blood pressure according to another embodiment. In the embodiment illustrated in Fig. 5, the device 100 is as described earlier with respect to Fig. 1. Fig. 6 is a simplified schematic illustration of the device 100 for use in measuring blood pressure according to yet another embodiment. In the embodiment illustrated in Fig. 6, the device 100 is as described earlier with respect to Fig. 3. However, whereas the light source 102 of device 100 described earlier with respect to Figs. 1 and 3 are illustrated as comprising a single light source, the light source 102 of the device 100 of each of the embodiments illustrated in Figs. 5 and 6 comprises a plurality of (e.g. separate) light sources 102a, 102b that are configured to alternate between emitting coherent light 104a and incoherent light 104b. In the embodiments illustrated in Figs. 5 and 6, at least one of the plurality of light sources 102a is configured to emit coherent light 104a and at least one other of the plurality of light sources 102b is configured to emit incoherent light 104b.

As illustrated in Figs. 5 and 6, in some embodiments, the at least one of the plurality of light sources 102a configured to emit coherent light 104a and the at least one other of the plurality of light sources 102b configured to emit incoherent light 104b may be positioned on the same side of the light detector 110 according to some embodiments or on opposite sides of the light detector 110 according to other embodiments. In other words, the light detector 110 can be positioned to one side of the plurality of light sources 102a, 102b according to some embodiments or between the at least one of the plurality of light sources 102a configured to emit coherent light 104a and the at least one other of the plurality of light sources 102b configured to emit incoherent light 104b according to other embodiments.

In the embodiments where the light detector 110 is positioned to one side of the plurality of light sources 102a, 102b in Figs. 5 and 6, the at least one of the plurality of light sources 102b configured to emit incoherent light 104b is illustrated as being closer to the light detector 110 than the at least one of the plurality of light sources 102a configured to emit coherent light 104a. However, it will be understood that the at least one of the plurality of light sources 102a configured to emit coherent light 104a may be positioned closer to the light detector 110 than the at least one of the plurality of light sources 102b configured to emit incoherent light 104b according to other embodiments.

In embodiments where at least two of the plurality of light sources 102a are configured to emit coherent light 104a, the light sources 102a configured to emit coherent 104a light can be synchronized in time. Similarly, in embodiments where at least two of the plurality of light sources 102a are configured to emit incoherent light 104b, the light sources 102a configured to emit incoherent light 104b can be synchronized in time.

Figs. 5 and 6 also illustrate the current of the plurality of light sources 102a, 102b according to some embodiments. In particular, as illustrated in Figs. 5 and 6, the current of the plurality of light sources 102a, 102b may be the same according to some embodiments. Thus, instead of altering the current of a single light source 102, in some embodiments, the plurality of light sources 102a, 102b may be configured to alternate between emitting coherent light 104a and incoherent light 104b by the at least one of the plurality of light sources 102a configured to emit coherent light ("C") 104a and the at least one other of the plurality of light sources 102b configured to emit incoherent light ("I") 104b being configured to emit light alternately. As illustrated in Figs. 5 and 6, the current ("Curr.") of the plurality of light sources 102a, 102b remains constant over time ("T").

Although not illustrated, in some of the embodiments illustrated in Fig. 5, the feedback loop described earlier with reference to Fig. 2 may be used from the light detector 110 to the current of the light source 102. Although also not illustrated, in some of the embodiments illustrated in Fig. 6, the feedback loop described earlier with reference to Fig. 4 may be used from the light detector 110 to the current of the light source 102.

As described earlier, in the embodiments described herein, the electrical signal generated when the light source 102 is configured to alternate between emitting coherent light and incoherent light is for use in measuring a blood pressure of the subject 108. In addition, in any of the embodiments described herein, the electrical signal generated when the light source 102 is configured to emit coherent light can be for use in measuring a blood flow in the skin 106 of the subject 108. In more detail, the light 104 scattered back from the skin 106 of the subject 108 can be scattered by both skin cells and blood cells. The blood cells are moving and thus the light scattered by the blood cells is Doppler shifted, whereas the light scattered by the skin is not Doppler shifted. When the scattered light 104 combines on the light detector 110, the Doppler shifted light interferes with the non-Doppler shifted light and thus the electrical signal generated by the detected light 104 comprises Doppler frequency components. In this way, the device 100 is capable of measuring the movement of the blood cells and is thus for use in measuring blood flow.

Thus, in some embodiments, the device 100 can be for use in measuring both blood pressure and blood flow. In this way, the blood pressure and blood flow can be measured at one (single) location using the same device 100. That is, the device 100 can enable local measurement of blood pressure and blood flow. This is possible, for example, since the coherent light and incoherent light probe (more or less) the same region of skin 106. As the light source 102 of the device 100 is configured to emit coherent light, the device 100 may be referred to as a laser Doppler flowmeter. However, the device 100 is improved by virtue of the light source 102 also being configured to emit incoherent light in the manner described herein. The device 100 may also be for use in measuring any other physiological parameters, such as one or more cardiovascular parameters (e.g. heart rate, arrhythmia, etc.).

In any of the embodiments described herein, a light source 102 may be configured to operate as a photodiode, e.g. a light emitting diode (LED), to emit incoherent light. In some embodiments, a light source 102 may be configured to operate as a laser (or a laser diode) to emit coherent light. In any of the embodiments described herein, the light source 102 may be configured to alternate between emitting coherent light and incoherent light with a predefined frequency. In some embodiments, the predefined frequency may be a frequency higher than that given by the pulse (e.g. in the Hz range) but lower than that necessary for laser Doppler measurements (e.g. in the kHz range).

For example, in some embodiments, the predefined frequency may be a frequency in a range from 10 Hz to 10 kHz, for example a frequency in a range from 100 Hz to 9 kHz, for example a frequency in a range from 200 Hz to 8 kHz, for example a frequency in a range from 300 Hz to 7 kHz, for example a frequency in a range from 400 Hz to 6 kHz, for example a frequency in a range from 500 Hz to 5 kHz, for example a frequency in a range from 600 Hz to 4 kHz, for example a frequency in a range from 700 Hz to 3 kHz, for example a frequency in a range from 800 Hz to 2 kHz, for example a frequency in a range from 900 Hz to 1 kHz. For example, in some embodiments, the predefined frequency may be a frequency of 10 Hz, 25 Hz, 525 Hz, 1025 Hz, 1525 Hz, 2025 Hz, 2525 Hz, 3025 Hz, 3525 Hz, 4025 Hz, 4525 Hz, 5025 Hz, 5525 Hz, 6025 Hz, 6525 Hz, 7025 Hz, 7525 Hz, 8025 Hz, 525 Hz, 9025 Hz, 9525 Hz, 10 kHz, or any integer or non-integer value between the mentioned values (e.g. 25 Hz).

Although not illustrated in Figs. 1-6, the device 100 may comprise a battery or other power supply for powering the device 100 or means for connecting the device 100 to a mains power supply. It will also be understood that the device 100 may comprise any other component to those described herein or any combination of components.

Although also not illustrated, there is further provided a system for use in measuring blood pressure. The system comprises the device as described earlier according to any of the embodiments. The system also comprises a processor. In some embodiments, the device 100 itself may comprise a processor. Alternatively or in addition, in some embodiments, a processor may be external to (e.g. separate to or remote from) the device 100. In these embodiments, the processor can be communicatively coupled to the device 100.

Although reference is made to "a" processor, it will be understood that the processor may comprise one or more processors. The one or more processors can be implemented in numerous ways, with software and/or hardware, to perform the various functions described herein. In some embodiments, each of the one or more processors can be configured to perform individual or multiple steps of the method described herein. In particular implementations, the one or more processors can comprise a plurality of software and/or hardware modules, each configured to perform, or that are for performing, individual or multiple steps of the method described herein. The one or more processors may comprise one or more microprocessors, one or more multi-core processors and/or one or more digital signal processors (DSPs), one or more processing units, and/or one or more controllers (such as one or more **microcontrollers**) that may be configured or programmed (e.g. using software or computer program code) to perform the various functions described herein. The one or more processors may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

In some embodiments, the processor can be configured to control the light source 102 of the device 100 to alternate between emitting coherent light and incoherent light. For example, the processor may be configured to control the current of the light source 102 in the manner described earlier. Alternatively or in addition, in some embodiments, the processor can be configured to process the electrical signal generated when the light source 102 alternates between emitting coherent light and incoherent light to measure the blood pressure of the subject 108. The processor can be configured to measure the blood pressure of the subject 108 in any suitable way.

For example, in some embodiments, the processor can be configured to determine a pulse amplitude of the electrical signal generated when the light source 102 emits incoherent light and determine a pulse wave velocity (PWV) of the electrical signal generated when the light source 102 emits coherent light. In these embodiments, the processor can be configured to measure the blood pressure of the subject 108 based on the determined pulse amplitude and the determined pulse wave velocity. For example, in some embodiments, the processor can be configured measure the blood pressure of the subject 108 based on the determined pulse amplitude and the determined pulse wave velocity using an equation (e.g. an analytical formula) or a table (e.g. a look-up table).

An example of an equation that may be used to measure the blood pressure of the subject 108 is, as follows:

$$P(t) - a * P_0 = b * \rho * PWV^2 ln\left(\frac{A(t)}{A_0}\right),$$

where $P(t)$ denotes the blood pressure as a function of time, $P_0$ denotes a starting pressure applied to the skin 106 of the subject 108 (e.g. measured with a pressure sensor), $t$ denotes time, $a$ and $b$ denote calibration parameters, $\rho$ denotes blood density (e.g. 1060 kg/m$^3$), $PWV$ denotes the determined pulse wave velocity, $A(t)$ denotes the determined pulse amplitude as a function of time, and $A_0$ denotes an initial pulse amplitude. In some embodiments, a calibration may be performed to determine the parameters $a$ and $b$. In an actuator embodiment, the actuator 116 can ensure that the pressure with which the device 100 is applied to the skin 106 of the subject 108 is maintained at the predefined pressure (e.g. a calibration pressure). The pressure can be maintained during the measurement of the blood pressure.

In some embodiments, the determined pulse amplitude of the electrical signal generated when the light source 102 emits incoherent light may be normalized. For example, a direct current (DC) component of the electrical signal generated when the light source 102 emits incoherent light may be determined. That is, the mean value of the electrical signal generated when the light source 102 emits incoherent light may be determined. The determined pulse amplitude may then be normalized by the value of this determined DC component. The determined pulse amplitude is related to blood volume. In particular, a variation in pulse amplitude corresponds to a variation in blood volume. The determined pulse wave velocity (PWV) of the electrical signal generated when the light source 102 emits coherent light is related to a (e.g. local) velocity of blood. In particular, a variation in pulse wave velocity corresponds to a variation in blood velocity. The blood velocity is the velocity of blood cells. More specifically, for example, the blood velocity can be the velocity of blood cells in the arterioles (rather than arteries).

In this way, the blood pressure of the subject 108 can be measured at any (or all) moments in time from the equation using the determined pulse amplitude and pulse wave velocity. The equation provides a continuous measurement of blood pressure. That is, the equation provides an instantaneous blood pressure as a function of time.

Alternatively or in addition to being configured to measure the blood pressure of the subject 108, in some embodiments, the processor can be configured to process the electrical signal generated when the light source 102 emits coherent light to measure the blood flow in the skin 106 of the subject 108. The electrical signal generated when the light source 102 emits coherent light provides an indication of the of the blood volume changes. More specifically, for example, the electrical signal generated when the light source 102 emits coherent light provides an indication of the of the blood volume changes of the arterioles in the skin (rather than arteries). The processor can be configured to measure the blood flow in the skin 106 of the subject 108 in any suitable way.

For example, in some embodiments, the processor can be configured to invert (or reverse) the electrical signal generated when the light source 102 emits coherent light and determine a maximum frequency of the inverted electrical signal. The inversion of this electrical signal provides an improved correlation. When there is more electrical signal, it means that there is less blood that absorbs the coherent light and thus less blood volume. The maximum frequency of the inverted electrical signal is related to the velocity of the blood. The maximum frequency of the inverted electrical signal may be determined in any suitable way. For example, in some embodiments, the processor can be configured determine the maximum frequency of the inverted electrical signal using an equation.

An example of an equation that may be used to determine the maximum frequency of the inverted electrical signal is, as follows:

$$f_{max}(t) = \frac{\int f \, S(f,t) \, df}{\int S(f,t) \, df},$$

where $f_{max}(t)$ denotes the maximum frequency of the inverted electrical signal as a function of time, $f$ denotes frequency, t denotes time, $S(f,t)$ denotes the electrical signal. Thus, the inverted electrical signal is integrated with respect to frequency to determine the maximum frequency of the inverted electrical signal.

In some embodiments, the processor can be configured to measure the blood flow in the skin 106 of the subject 108 as an average velocity of blood flow in the skin 106 of the subject 108. In some of these embodiments, the average velocity of blood flow (e.g. of moving blood cells) in the skin 106 of the subject 108 may be half of a product of the determined maximum frequency of the inverted electrical signal and a wavelength at which the coherent light is emitted. For example, in some embodiments, the processor can be configured determine average velocity of blood flow in the skin 106 of the subject 108 using an equation, such as the following Doppler equation:

$$v(t) = \frac{\lambda f_{max}(t)}{2},$$

where $v(t)$ denotes the average velocity of blood flow in the skin 106 of the subject 108 as a function of time, $\lambda$ denotes the wavelength at which the coherent light is emitted, and $f_{max}(t)$ denotes the determined maximum frequency of the inverted electrical signal as a function of time.

As mentioned earlier, the electrical signal generated by the detected light 104 comprises Doppler frequency components. As the Doppler frequency components are generally in the order of kHz, in some embodiments, the processor can be configured to process only an alternating current (AC) component of the generated electrical signal when the light source 102 emits coherent light to measure the blood flow in the skin 106 of the subject 108. That is, in some embodiments, the DC component of the generated electrical signal is not used by the processor in measuring the blood flow in the skin 106 of the subject 108, even though traces of the Doppler frequency components may be present in it, at very low frequencies (e.g. 1 Hz). For example, the traces may be noisy and thus the quality of the signal may be low using the DC component of the generated electrical signal for the blood flow measurement. Thus, by using the AC component of the generated electrical signal for the blood flow measurement, the blood flow can be more accurately measured.

In some embodiments, the system may comprise a communications interface (or communications circuitry). In some embodiments, the device 100 described earlier may comprise a communications interface. Alternatively or in addition, the communications interface may be external to (e.g. separate to or remote from) the device 100. The communications interface can be for enabling the device 100, or components of the device 100, to communicate with and/or connect to one or more other components (e.g. one or more sensors, interfaces, devices, processors, or memories), such as any of those described herein. For example, the communications interface can be for enabling the device 100 (or one or more components of the device 100, such as the light source 102 and/or the light detector 110) to communicate with and/or connect to a processor (such as that mentioned earlier).

The communications interface may enable the device 100, or components of the device 100, to communicate and/or connect in any suitable way. For example, the communications interface may enable the device 100, or components of the device 100, to communicate and/or connect wirelessly, via a wired connection, or via any other communication (or data transfer) mechanism. In some wireless embodiments, for example, the communications interface may enable the device 100, or components of the device 100, to use radio frequency (RF), Bluetooth, or any other wireless communication technology to communicate and/or connect.

In some embodiments, the system may comprise at least one memory. In some embodiments, the device 100 described earlier may comprise at least one memory. Alternatively or in addition, at least one memory may be external to (e.g. separate to or remote from) the device 100. For example, a hospital database may comprise the memory, the memory may be a cloud computing resource, or similar. The device 100 (or a processor of the system) may be configured to communicate with and/or connect to at least one memory. A memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory can be configured to store program code that can be executed by a processor (such as a processor of the device 100) to cause the device 100 to operate in the manner described herein.

Alternatively or in addition, in some embodiments, at least one memory can be configured to store information required by or resulting from the method described herein. For example, in some embodiments, at least one memory may be configured to store any one or more of the electrical signal generated from the detected light 104, a measured blood pressure of the subject 108, a measured blood flow in the skin 106 of the subject 108, or any other information, or any combination of information, required by or resulting from the method described herein. In some embodiments, the device 100 (or a processor of the system) can be configured to control at least one memory to store information required by or resulting from the method described herein.

The system may comprise a user interface. In some embodiments, the device 100 described earlier may comprise the user interface. Alternatively or in addition, the user interface may be external to (e.g. separate to or remote from) the device 100. The device 100 (or a processor of the system) may be configured to communicate with and/or connect to a user interface. In some embodiments, the device 100 (or a processor of the system) can be configured to control the user interface to operate in the manner described herein.

The user interface can be configured to render (or output, display, or provide) information required by or resulting from the method described herein. For example, in some embodiments, the user interface may be configured to render (or output, display, or provide) one or more of the electrical signal generated from the detected light 104, a measured blood pressure of the subject 108, a measured blood flow in the skin 106 of the subject 108, or any other information, or any combination of information, required by or resulting from the method described herein. Alternatively or in addition, the user interface can be configured to receive a user input. For example, the user interface may allow a user to manually enter information or instructions, interact with and/or control the device 100. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

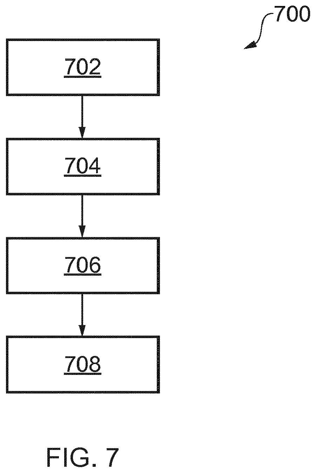

Fig. 7 is a flow chart illustrating a method 700 of operating a device 100 according to an embodiment. The device 100 is as described earlier with reference to the embodiments illustrated in any of Figs. 1-6. With reference to Fig. 7, at block 702 of Fig. 7, the light 104 is emitted onto the skin 106 of the subject 108 in the manner described earlier. At block 704 of Fig. 7, the light source 102 is alternated between emitting coherent light and incoherent light in the manner described earlier. At block 706 of Fig. 7, the light 104 scattered back from the skin of the subject 108 is detected in the manner described earlier. At block 708 of Fig. 7, the electrical signal for use in measuring a blood pressure of the subject 108 is generated from the detected light in the manner described earlier.

Although not illustrated in Fig. 7, in some embodiments, the method 700 may further comprise processing the electrical signal generated when the light source 102 alternates between emitting coherent light and incoherent light to measure the blood pressure of the subject 108 in the manner described earlier. Alternatively or in addition, in some embodiments, the method 700 may further comprise processing the electrical signal generated when the light source 102 emits coherent light to measure the blood flow in the skin 106 of the subject 108 in the manner described earlier.

There is also described a computer program product comprising a computer readable medium. The computer readable medium has a computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk).

Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein a device, a system, a method and a computer program product that address the limitation associated with the existing techniques. Therefore, there is provided an improved technique for use in measuring blood pressure and optionally also blood flow.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A device (100) for use in measuring blood pressure, wherein the device (100) comprises:

   a light source (102, 102a, 102b) configured to emit light (104, 104a, 104b) onto a portion of the skin (106) of a subject (108); and
   a light detector (110) configured to detect light (104, 104a, 104b) scattered back from within the portion of the skin (106) of the subject (108) and generate an electrical signal from the detected light (104, 104a, 104b);
   wherein the light source (102, 102a, 102b) is configured to alternate between emitting coherent light (104a) and incoherent light (104b) and the generated electrical signal is for use in measuring a blood pressure of the subject (108).

2. The device (100) as claimed in claim 1, wherein the light source (102) is configured to alternate between emitting coherent light and incoherent light when a current of the light source (102) is altered.

3. The device (100) as claimed in any of the preceding claims, wherein the light source (102) is configured to:

   emit coherent light when the current of the light source (102) is increased above a threshold current; and/or
   emit incoherent light when the current of the light source (102) is decreased below the threshold current.

4. The device (100) as claimed in claim 3, wherein the light source (102) is configured to:

   emit coherent light when the current of the light source (102) is increased to a current that is a predefined amount greater than the threshold current; and/or
   emit incoherent light when the current of the light source (102) is decreased to a current that is the predefined amount less than the threshold current.

5. The device (100) as claimed in any of the preceding claims, wherein the light source (102, 102a, 102b) is configured to alternate between emitting coherent light (104a) and incoherent light (104b) with a predefined frequency.

6. The device (100) as claimed in any of the preceding claims, wherein:
   the electrical signal generated when the light source (102, 102a) is configured to emit coherent light (104a) is for use in measuring a blood flow in the skin (106) of the subject (108).

7. The device (100) as claimed in any of the preceding claims, wherein the light source (102, 102b) is configured to operate as a light emitting diode to emit incoherent light (104b).

8. The device (100) as claimed in any of the preceding claims, wherein the light source (102, 102a) is configured to operate as a laser to emit coherent light (104a).

9. The device (100) as claimed in any of the preceding claims, wherein the device (100) further comprises: an actuator (116, 116a, 116b) configured to maintain a pressure with which the device (100) is applied to the skin (106) of the subject (108) at a predefined pressure.

10. A system for use in measuring blood pressure, the system comprising:

the device (100) as claimed in any of the preceding claims; and
a processor configured to:

process the electrical signal generated when the light source (102, 102a, 102b) alternates between emitting coherent light (104a) and incoherent light (104b) to measure the blood pressure of the subject (108); and/or
process the electrical signal generated when the light source (102, 102a) emits coherent light (104a) to measure the blood flow in the skin (106) of the subject (108).

11. The system as claimed in claim 10, wherein the processor is configured to measure the blood pressure of the subject (108) by being configured to:

determine a pulse amplitude of the electrical signal generated when the light source (102, 102b) emits incoherent light (104b);
determine a pulse wave velocity of the electrical signal generated when the light source (102, 102a) emits coherent light (104a); and
measure the blood pressure of the subject (108) based on the determined pulse amplitude and the determined pulse wave velocity.

12. The system as claimed in claim 10 or 11, wherein the processor is configured to measure the blood flow in the skin (106) of the subject (108) by being configured to:

invert the electrical signal generated when the light source (102, 102a) emits coherent light (104a);
determine a maximum frequency of the inverted electrical signal; and
measure the blood flow in the skin (106) of the subject (108) as an average velocity of blood flow in the skin (106) of the subject (108), wherein the average velocity of blood flow in the skin (106) of the subject (108) is half of a product of the determined maximum frequency of the inverted electrical signal and a wavelength at which the coherent light (104a) is emitted.

13. A method (700) of operating a device (100) as claimed in any of claims 1 to 9, wherein the method (700) comprises:

emitting (702) the light (104, 104a, 104b) onto a portion of the skin (106) of the subject (108);
alternating (704) the light source (102, 102a, 102b) between emitting coherent light (104a) and incoherent light (104b);
detecting (706) the light (104, 104a, 104b) scattered back from within said portion of the skin (106) of the subject (108); and
generating (708) the electrical signal from the detected light (104, 104a, 104b).

14. The method (700) as claimed in claim 13, further comprising:

processing the electrical signal generated when the light source (102, 102a, 102b) alternates between emitting coherent light (104a) and incoherent light (104b) to measure the blood pressure of the subject (108); and/or
processing the electrical signal generated when the light source (102, 102a) emits coherent light (104a) to measure the blood flow in the skin (106) of the subject (108).

15. A computer program product comprising a computer readable medium, the computer readable medium having a computer readable code embodied therein, the computer readable code being configured such that, on execution by a suitable computer or processor, the computer or processor is caused to perform the method as claimed in claim 13 or 14.

FIG. 1

FIG. 2

FIG. 3

FIG. 4

FIG. 5

FIG. 6

700

702

704

706

708

FIG. 7

Europäisches
Patentamt

European
Patent Office

Office européen
des brevets

# EUROPEAN SEARCH REPORT

Application Number

EP 18 20 9838

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | US 2017/231513 A1 (PRESURA CRISTIAN NICOLAE [NL] ET AL)<br>17 August 2017 (2017-08-17)<br>* paragraphs [0030], [0032], [0034], [0036], [0038], [0041] - [0042], [0048], [0049] *<br>* figures 1, 4a-c, 5 * | 1-4,<br>6-10,<br>13-15<br>11,12 | INV.<br>A61B5/021<br>A61B5/026<br>A61B5/00 |
| X | US 2016/220129 A1 (OSTROVERKHOV VICTOR PETROVICH [US] ET AL)<br>4 August 2016 (2016-08-04)<br>* paragraphs [0071] - [0077], [0087], [0092] *<br>* figures 6, 8-10 * | 1,2,6-8,<br>10,13-15 | |
| X | BOGGETT ET AL: "Laser doppler measurements of blood flow in skin tissue",<br>JOURNAL OF BIOMEDICAL ENGINEERING, BUTTERWORTH, GUILDFORD, GB,<br>vol. 7, no. 3, 1 July 1985 (1985-07-01), pages 225-232, XP022445151,<br>ISSN: 0141-5425, DOI:<br>10.1016/0141-5425(85)90023-8<br>* page 226 - page 228 *<br>* figure 1 * | 1,2,13,<br>15 | |
| X | US 2016/242657 A1 (WANG ROBERT CHENG-YUAN [US] ET AL) 25 August 2016 (2016-08-25)<br>* paragraphs [0031] - [0041] *<br>* figures 1-3 * | 1,2,5,<br>13,15 | |
| Y | EP 0 443 267 A1 (SENTINEL MONITORING INC [US]) 28 August 1991 (1991-08-28)<br>* column 3, lines 10-30 * | 11 | |
| Y | US 9 962 075 B2 (UNIV NORTHWESTERN [US]; OPTICENT INC [US]) 8 May 2018 (2018-05-08)<br>* column 19, lines 7-30 * | 12 | |

TECHNICAL FIELDS
SEARCHED (IPC)

A61B

The present search report has been drawn up for all claims

| Place of search | Date of completion of the search | Examiner |
|---|---|---|
| The Hague | 3 May 2019 | Trattner, Barbara |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 20 9838

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2019

| Patent document cited in search report | | Publication date | Patent family member(s) | | | Publication date |
|---|---|---|---|---|---|---|
| US 2017231513 | A1 | 17-08-2017 | CN | 105979860 | A | 28-09-2016 |
| | | | EP | 3076861 | A1 | 12-10-2016 |
| | | | JP | 6077189 | B1 | 08-02-2017 |
| | | | JP | 2017505660 | A | 23-02-2017 |
| | | | US | 2017231513 | A1 | 17-08-2017 |
| | | | WO | 2016037991 | A1 | 17-03-2016 |
| US 2016220129 | A1 | 04-08-2016 | NONE | | | |
| US 2016242657 | A1 | 25-08-2016 | NONE | | | |
| EP 0443267 | A1 | 28-08-1991 | CA | 2036900 | A1 | 24-08-1991 |
| | | | EP | 0443267 | A1 | 28-08-1991 |
| | | | JP | H0614891 | A | 25-01-1994 |
| US 9962075 | B2 | 08-05-2018 | AU | 2015253295 | A1 | 15-12-2016 |
| | | | CA | 2950102 | A1 | 05-11-2015 |
| | | | CN | 106455972 | A | 22-02-2017 |
| | | | EP | 3136950 | A1 | 08-03-2017 |
| | | | US | 2015348287 | A1 | 03-12-2015 |
| | | | US | 2017188818 | A1 | 06-07-2017 |
| | | | US | 2018256025 | A1 | 13-09-2018 |
| | | | WO | 2015168157 | A1 | 05-11-2015 |

For more details about this annex : see Official Journal of the European Patent Office, No. 12/82

**EP 3 662 821 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007017661 A